# EUROPEAN PATENT APPLICATION

(11) **EP 1 317 935 A1**
(43) Date of publication of application: **11.06.2003**
(21) Application number: 02396179.0
(22) Date of filing: 02.12.2002
(51) Int. Cl.: A61L 31/14, A61L 27/50, A61L 27/58, A61L 27/56

(54) **Biodegradable implant and method for manufacturing one**

(30) Priority: 04.12.2001 US 6796
(71) Applicant: Inion Ltd., 33520 Tampere (FI)
(72) Inventor: Pirhonen, Eija, 33100 Tampere (FI); Nieuwenhuis, Jan, 4207 KD Gorinchen (NL); Pohjonen, Timo, 33710 Tampere (FI)
(74) Representative: Kaukonen, Juha Veikko

(57) **Abstract**

A biodegradable implant and a method for manufacturing one. The implant comprises a matrix component containing at least one biodegradable polymer or copolymer and a plasticizer that is adapted to substantially reduce the rigidity of the implant. The plasticizer substantially exists from the implant after coming into contact with tissue fluids of the organ system in such a manner that the bending resistance of the implant prior to the insertion of the implant into the organ system is substantially lower than after its insertion into the organ system.

## Description

### FIELD OF THE INVENTION

The invention relates to a biodegradable implant that comprises a matrix component containing at least one biodegradable polymer or copolymer.

Further, the invention relates to a method for manufacturing a biodegradable implant, in which method the matrix component of the implant is formed by selected biodegradable polymers or copolymers.

### BACKGROUND OF THE INVENTION

The invention relates to artificial implants that are made of biodegradable materials and can be implanted in the organ system. Herein, the concept 'implant' refers to shaped pieces to be implanted in the organ system, such as membranes, fixation plates, other three-dimensional spatial pieces, fixing means, such as screws, pins and sutures, and the like, that are used to support or attach tissue or to separate tissue from other tissue while healing. The invention also relates to guided tissue regeneration (GTR), and herein especially to applying artificial membranes to strengthen insufficient tissue, regenerate missing tissue, and regenerate lost tissue, and the like.

Certain surgical interventions, for instance, entail a need to regenerate tissue in a certain, controlled manner. The reason for the intervention can be for instance a fractured bone, the regeneration of new tissue to replace tissue lost due to traumatic or surgical causes or an infection, atrophy or congenital reasons. In most cases, a successful performance of such interventions requires that the affected tissue be separated from other tissues surrounding it and that around the tissue a certain space is created, to which tissue can regenerate. This is generally done by a membrane implant, called membrane in the following.

In odontology, a typical application example of GTR in regeneration of tissue is separating an inflamed root of a tooth by a membrane from the connective tissue and epithelium of the gum. A periodontal ligament tissue and dental cement tissue can grow on the surface of the root and a new connective tissue attachment is formed to renew the attachment of the root of the tooth. The separation is necessary, since connective tissue and epithelium grow naturally faster than the ligament and dental cement tissue; in other words, if the root of the tooth is not separated, the epithelium or the connective tissue of the gum grows on the surface of the root and no periodontal ligament and dental cement tissue can be formed. This way, the root attached itself poorly to the surrounding tissue. Instead, the membrane prevents the connective tissue and epithelium from growing on the surface of the root. At the same time, it creates a space for the periodontal ligament and dental cement to form. The root then attaches firmly to the surrounding tissue.

Essential properties of the membrane are its shapability and rigidity. Namely, the membrane must naturally be shaped to fit the tissue structure in such a manner that it separates the tissues from each other exactly as desired so as to allow the regenerating tissue to grow in the correct shape with no damage to the surrounding tissue. On the other hand, the membrane must be sufficiently rigid that its shape does not change under the pressure caused by the growing tissue or that possible external stress does not cause a movement hampering the healing of the tissue. A sufficient rigidity of the membrane is thus a requirement for the tissue to actually grow to the desired shape and size. The essential properties of the membrane are contrary to each other. Prior art does not provide a satisfactory solution to fulfilling both requirements.

Bio-stable membranes are known that are made of GoreTex, for instance, i.e. a porous polytetrafluoroethylene (PTFE), and whose rigidity is increased by titanium support threads. Such membranes are often rigid and therefore keep their form well under the pressure of tissue, but correspondingly, their shaping is arduous. Shaping support thread-free PTFE membranes is quite easy, but their rigidity is not sufficient. A second significant problem with these membranes is that they must be removed surgically from the organ system after the tissue has healed. Surgical removal means costs, discomfort to the patient and adds to the patient's risk of obtaining an infection from the operation, for instance.

Membranes dissolving in the organ system, i.e. membranes made of biodegradable polymers, are also known. Such membranes need not be surgically removed from the organ system, but they exit slowly from the organ system with the normal metabolism of the person. The problem with biodegradable materials is that the thin, easily shaping membranes are not rigid enough to maintain space for the regenerating tissue to grow undisturbed. The risk is then significantly high that the membrane bends under pressure against the healing tissue in such a manner that there is not enough space for the regenerating tissue to form. To achieve a sufficient rigidity, the membrane can naturally be made thicker. When the thickness of the membrane is increased to achieve a sufficient rigidity, the membrane becomes so thick that shaping it is very difficult and arduous.

US patent 5,919,234 discloses a porous and flexible membrane that in one of its embodiments is entirely biodegradable. The structure of the membrane is a compromise between shapability and rigidity so that both requirements are to some extent met. The shapability and/or rigidity of the disclosed membrane are, however, not optimal.

US patent 5,525,646 discloses a biodegradable material and a product made thereof. The pursued material properties were on one hand good shapability and on the other hand dimensional stability. The material comprises a biodegradable amorphous polymer section, a biodegradable crystalline polymer section and a plasticizer. The amorphous polymer section is selected from the following group: poly(D,L-lactide), poly(D,L-lactide) and its copolymer with polycaprolactone, poly(L-lactide) or polytrimethylcarbonate. The crystalline polymer section is selected from a group including poly(L-lactide), polycaprolactone and polydioxanone. The plasticizer is selected from the following group: ethyl, butyl or capryl ester of acetylated citric acid and ethyl-terminated oligomers of lactic acid having the length of 2 to 10 lactic acid units. The solution is a compromise between the shapability and rigidity of a membrane made of the material, but does not optimize the rigidity and shapability of the membrane.

WO publication 96/34634 discloses a membrane applied to guided tissue regeneration, the body of the membrane being a textile fabric woven or knitted from biodegradable fibers. The textile fabric is coated with a solution containing a biodegradable polymer and a micropore-generating agent. The material of the textile fabric is polylactide or polyglycolide. The polymer solution contains for instance polylactide, polyglycolide or polytrimethylcarbonate, and an N-methyl-2-pyrrolidone (NMP) solution, to which the polymer has been dissolved. The stability and shapability of the membrane is improved by thermal treatment of the membrane. The structure of the membrane is complex and thus also expensive. In addition, the thermal treatment is an extra manufacturing stage adding to the costs.

Other types of implants are fixation plates used in osteosynthesis to support a healing bone. Appropriate fitting together of bone parts requires that the fixation plate can be shaped exactly according to the shape of the bone parts to be fitted together. The fixation plate should be flexible between the fixing holes and capable of encompassing the shapes of the bone parts. The shaping is, however, arduous, because the bending and twisting resistances of the fixation plate parts between the fixing holes are great, and a lot of force is required in the shaping - even though pliers and clamps are used in it. Shaping the fixation plate causes a lot of work during the operation, thus extending the operation time and causing extra costs. In addition, the fitting of the fixation plate may remain insufficient, which prevents its use in the target of application, or if the poorly fitting fixation plate for one reason or another is operated to the bone, it can at worst impede an appropriate healing of the bone.

### BRIEF DESCRIPTION OF THE INVENTION

It is an object of the present invention to provide a novel and improved biodegradable implant that is easy and effortless to shape into the required shape and that is still sufficiently rigid to support or attach tissues in a desired manner when in place. An object of the invention is also to provide a novel and improved method for manufacturing a biodegradable implant, which method is simple, effortless and inexpensive.

The implant of the invention is characterized in that it comprises a plasticizer that is adapted to reduce substantially the rigidity of the implant and that substantially exits from the implant after coming into contact with tissue fluids of the organ system in such a manner that the bending resistance of the implant prior to the insertion of the implant into the organ system is substantially lower than after its insertion into the organ system.

Further, the method of the invention is characterized by adding to the matrix component a plasticizer that substantially exits from the implant after coming into contact with tissue fluids of the organ system in such a manner that the rigidity of the implant increases substantially after the insertion of the implant into the organ system.

The essential idea of the invention is that the implant comprises a plasticizer that exits from the implant after its insertion into the organ system to such an extent that the bending resistance of the implant increases substantially from what it was just before the implant was inserted into the organ system. Further, the essential idea of a preferred embodiment of the invention is that the plasticizer is substantially N-methyl-2-pyrrolidone (NMP). Further, the essential idea of a second preferred embodiment of the invention is that the plasticizer is added to the matrix material at the latest at the forming stage of the implant. Further, the essential idea of a third preferred embodiment of the invention is that the plasticizer is added to the implant just before the membrane is inserted into the organ system.

The invention provides the advantage that the rigidity of the implant prior to the insertion of the implant into the organ system is substantially less than after the insertion. Thus, the implant can at its shaping stage very easily be shaped into the required shape and yet its rigidity is sufficient to support or attach the tissue during healing. Yet another advantage of the invention is that when the plasticizer diffuses from the implant, a porous layer is formed on the outer surfaces of the implant that serves as a structure guiding the regeneration of the tissue and aids the attaching of the tissue to the implant.

### BRIEF DESCRIPTION OF THE FIGURES

The invention is described in more detail in the attached drawings, in which
Figure 1a is a schematic representation of a periodontal defect,
Figure 1b is a schematic representation of an implant of the invention fitted over the defect shown in Figure 1a,
Figure 2a is a schematic representation of a bone defect site in the bony tissue surrounding a dental alveolus,
Figure 2b shows a second implant of the invention fitted over the bone defect site shown in Figure 2a,
Figure 3 is a schematic representation of the surface of an implant of the invention as a SEM figure, and
Figure 4 is a schematic representation of a third implant of the invention from the direction of its top surface.

### DETAILED DESCRIPTION OF THE INVENTION

It should be noted that even though the following figures and examples describe the invention using membranes and fixation plates, the invention could be applied in a corresponding manner to other shaped biodegradable pieces to be inserted into the organ system, such as three-dimensional spatial pieces and fixing means.

The matrix material of an implant of the invention can comprise for instance the following biodegradable polymers: polyglycolide, polylactides, polycaprolactones, polytrimethylenecarbonates, polyhydroxybutyrates, polyhydroxyvalerates, polydioxanones, polyorthoesters, polycarbonates, polytyrosinecarbonates, polyorthocarbonates, polyalkylene oxalates, polyalkylene succinates, poly(malic acid), poly(maleic anhydride), polypeptides, polydepsipeptides, polyvinylalcohol, polyesteramides, polyamides, polyanhydrides, polyurethanes, polyphosphazenes, polycyanoacrylates, polyfumarates, poly(amino acids), modified polysaccharides (like cellulose, starch, dextran, chitin, chitosan, etc.), modified proteins (like collagen, casein, fibrin, etc.) and their copolymers or combinations or mixtures or polymer blends thereof. Preferred polymers are polyglycolide, poly(L-lactide-co-glycolide), poly(D,L-lactide-co-glycolide), poly(L-lactide), poly(D,L-lactide), poly(L-lactide-co-D,L-lactide), polycaprolactone, poly(L-lactide-co-caprolactone), poly(D,L-lactide-co-caprolactone) polytrimethylenecarbonate, poly(L-lactide-co-trimethylenecarbonate), poly(D,L-lactide-co-trimethylenecarbonate), polydioxanone and copolymers and polymer blends of thereof. Suitable biodegradable polymers, copolymers and polymer mixtures are listed for instance in the following publications:
"Encyclopedic Handbook of Biomaterials and Bioengineering, Part A"
Donald L. Wise, Debra J. Trantolo, David E. Altobelli, Michael J. Yaszemski, Joseph D. Gresser, Edith R. Schwartz. 1992 by Marcel Dekker, Inc., pages 977 to 1007,
"Biodegradable fracture-fixation devices in maxillofacial surgery",
R. Suuronen. Int. J. Oral Maxillofac. Surg. 1993; 22: 50-57,
"Critical Concepts of Absorbable Internal Fixation",
William S. Pietrzak. Portland Bone Symposium, Portland, Oregon, August 4-7, 1999,
"High-impact poly(L/D-lactide) for fracture fixation: in vitro degradation and animal pilot study",
Jan Tams, Cornelis A.P. Joziasse, Ruud R.M. Bos, Fred R. Rozema, Dirk W. Grijpma and Albert J. Pennings. Biomaterials 1995, Vol. 16 No. 18, pages 1409 to 1415,
"A Review of Material Properties of Biodegradable and Bioresorbable Polymers and Devices for GTR and GBR Applications",
Dietmar Hutmacher, Markus B.Hürzeler, Henning Schliephake. The International Journal of Oral & Maxillofacial Implants. Volume 11, Number 5, 1996, pages 667 to 678, and
"Orthopaedic Application for PLA-Pga Biodegradable Polymers",
Kyriacos A. Athanasiou, Mauli Agrawal, Alan Barber, Stephen S. Burkkhart. The Journal of Arthroscopic and Related Surgery, Vol. 14, No 7 (October), 1988; 726 to 737.

Further, the matrix component can be a so-called combination material, i.e. composite, that can contain bio-glass, bio-ceramics, a pharmaceutical product, such as an antibiotic or growth factor, etc.

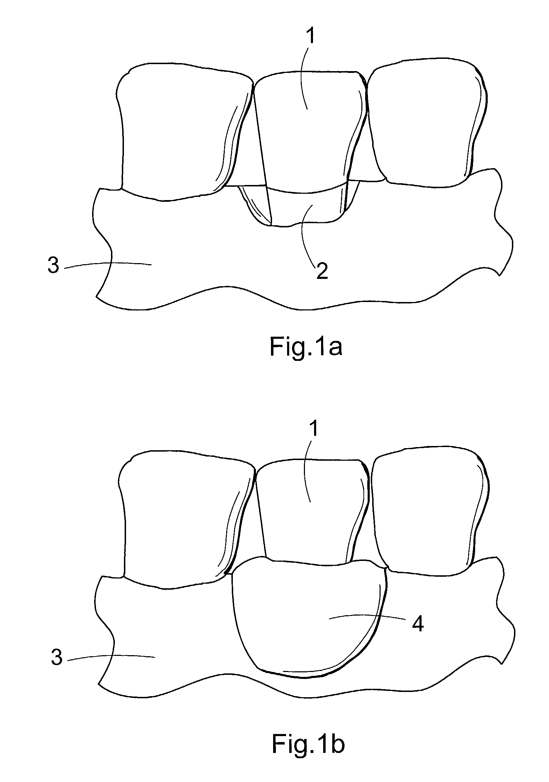

Figures 1a and 1b show a membrane implant of the invention applied to separate the root of a tooth from the connective tissue and epithelium of the gum. The inflamed root 2 of the tooth 1 has been cleaned surgically in Figure 1a. Figure 1b shows a membrane 3 that is cut and bent to a suitable shape around the cleaned root 2 and fixed in place by tissue adhesive, suture or pins. The membrane 3 separates the root 2 from the surrounding connective tissue and epithelium of the gum, thus preventing them from growing to the surface of the root 2. It should be noted that to simplify the description, Figures 1a and 1b do not show the gum tissues. The membrane also creates a space, to which the regenerating bony tissue of the jawbone 4 grows and which enables the periodontal ligament tissue and dental cement tissue to grow on the surface of the root 2.

The membrane also comprises a plasticizer that is N-methyl-2-pyrrolidone (NMP). The use of NMP is described in the above-mentioned WO publication 96/34634 and in the article "Preliminary *In Vivo* Studies on the Osteogenic Potential of Bone Morphogenetic Proteins Delivered from an Absorbable Puttylike Polymer Matrix"; Kirk P. Andriano, Bhagya Chandrashekar, Kathleen McEnery, Richard L. Dunn, Katie Moyer, Catherine M. Balliu, Kathleen M. Holland, Steven Garrett, William E. Huffer - J Biomed Mater Res (Appl Biomater) 53: 36-43, 2000. The article discloses a material containing poly(D,L-lactide-co-glycolid) dissolved in NMP and having its normal hydroxyl and ester end-groups replaced by hydroxy and carboxyl end-groups. This resulted in a mixture having a polymer content of 60 percent by weight and a viscosity that is so low that the mixture could be injected through an injection needle.

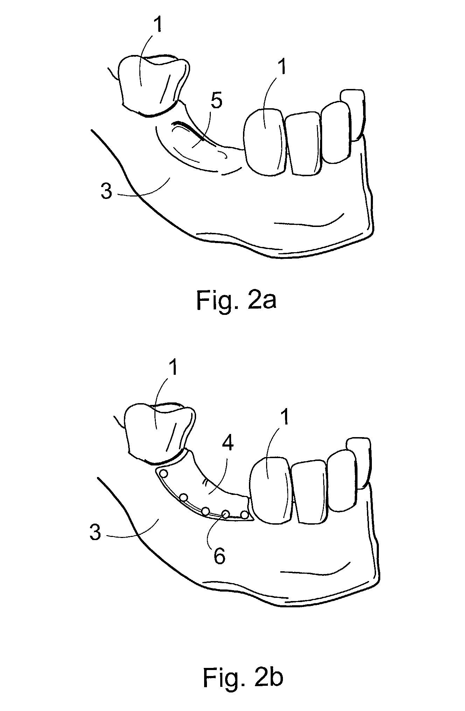

Figure 2a is a schematic representation of a bone defect site in the bone surrounding a dental alveolus, and Figure 2b shows a second implant of the invention fitted over said bone defect site. There is a need to create more bony tissue in the defect site 5 for instance in order to a tooth implant could be attached to the alveolus. The bone defect site 5 is filled with a osteinductive or osteoconductive material, such as bioactive glass particle mass, tricalciumphosphate, hydroxyapatite or the like, after which a membrane 4 is fixed over the defect site 5. In the embodiment shown in the figure, the membrane 4 is fixed by pins 6 made of a biodegradable material to the jawbone 3. Of course, the membrane 4 can also be fixed in any other manner known per se.

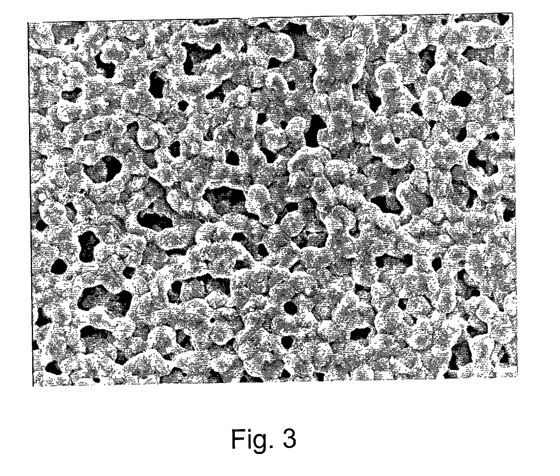

Figure 3 is a schematic representation of the surface of an implant of the invention as a SEM figure. The surface of the implant is enlarged 500fold. The implant in question is a GTR membrane made of poly(L-lactide-co-trimethylencarbonate-co-polyglycolide)-copolymer (PLLA/TMC/PGA) 40:20:40 according to example 3. After immersion in NMP, the membrane is dried in indoor air. The porosity of the surface of the implant is clearly visible. It is preferable that the implant is porous at least on its surface, because regenerating tissue attaches more firmly to a porous implant surface than to an even surface. Pores are created on the surface of the implant of the invention when NMP dissolves or evaporates from the implant. The pores are in the range of 10 µm in size. Pores can also be created on the implant in other known ways, such as by means of soluble salts or gases. The article "Bone regeneration with resorbable polymeric membranes. III. Effect of poly(L-lactide) membrane pore size on the bone healing process in large defects"; Leonilo M. Pineda, Michael Büsing, Richard P. Meinig, and Sylwester Gogolewski - Journal of Biomedical Materials Research, Vol. 31, 385 - 394 (1996) - discloses the use of a membrane made of poly(L-lactide) and the significance of the porosity of the membrane in repairing a defect in the tibia. It is preferable to apply the membrane of the invention to such a target, because the membrane is easy to shape to its correct shape and yet when inserted in the organ system, its rigidity is sufficiently high. Naturally, a membrane-shaped implant of the invention can also be applied to other surgical interventions. The membrane can be used for instance to support and shape bioactive glass particle masses, tricalciumphosphate, hydroxyapatite or the like used as filling agents for bony tissue as shown in figure 2b.

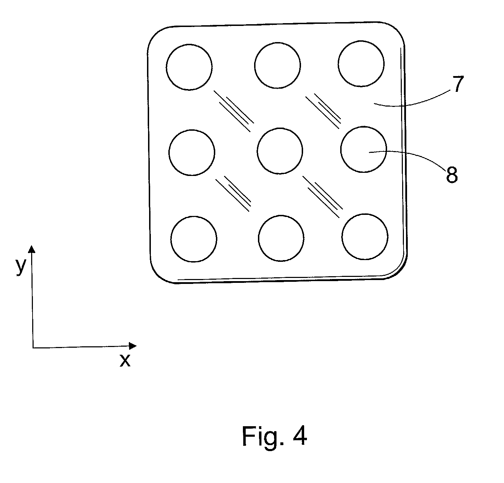

Figure 4 is a schematic representation of a third implant of the invention from the direction of its top surface. Here, the implant is a fixation plate 7. The fixation plate is fixed on both sides of the fractured or splintered point of the bony tissue using fixing elements, such as screws or pins, fitted through fixing holes 8. The fixation plate 7 keeps the bone in the correct position to allow it to heal in the best possible manner. The fixation plate 7 shown in Figure 4 comprises a total of nine fixing holes 8 in rows extending along both the X- and the Y-axis. Naturally, the fixing holes 8 can also be located in the fixation plate in other ways: they can be arranged diagonally in relation to the edge of the fixation plate 7 or they can be arranged in a subgroup of a certain shape, and these subgroups are then fitted in the implant in a suitable order, or in any other manner known per se.

The fixation plate of the invention can be an elongated rod, shaped like the letter L, T, X or Y, curved in a certain manner or a fixation plate of any other shape known per se, made of a matrix component comprising a biodegradable polymer or copolymer and a plasticizer, preferably NMP, added to it. Due to the plasticizer, the fixation plate is easy to shape in an optimal shape. When the fixation plate is inserted in the organ system, it comes into contact with tissue fluids and diffuses from the fixation plate, which increases substantially the rigidity of the sheet. The fixation plate of the invention is thus easy to shape and yet supports the healing bone sufficiently. The invention will be described in more detail in the following working examples.

### Example 1

A blank was made by extrusion of trimethylencarbonate/polylactide copolymer TMC/PLA (10:90), and the blank was further worked by compression molding into a 0.2-mm thick membrane. The highest used compression pressure was 100 bar and the maximum temperature was 180°C.

Strips of 80 mm in length and 10 mm in width were cut from the membrane for a tensile test. Four sets of strips, A, B, C and D, were randomly selected, each set having five strips.

The samples of sets A and B were immersed in an NMP solution and kept there for 30 seconds. After this, the samples were lifted from the solution and placed on top of a metal net for 30 minutes to ensure the diffusion of NMP to the polymer. The method describes a preferred embodiment of the invention, in which the implant is treated with NMP just before it is inserted in place in the patient.

The samples were tested with a generally known Instron material testing device. The testing was done according to the SFS-ISO 1184 standard. The pulling rate was 20 mm/min and the distance between the sample holders in the initial state was 50 mm. The samples of sets A and C were tested at indoor temperature 20°C without any special pre-treatment. The samples of sets B and D were instead kept in a water bath of 37°C for 24 hours before testing. The water bath treatment represents the situation where the samples or membranes are placed in the organ system of a patient. The tensile test of the samples of sets A and C was conducted in indoor air and the tensile test of the samples of sets B and D in a water bath of 37°C. The results of the tensile test are shown in table 1.

**Table 1:**

| Yield strength and tensile modulus averages of the tested sets | | | |
|---|---|---|---|
| Set | Test conditions | Yield strength [MPa] | Tensile modulus [MPa] |
| A (NMP) | Indoor air | 2.41 | 26.35 |
| B (NMP) | Water bath 37°C | 5.34 | 57.48 |
| C | Indoor air | 55.18 | 153.00 |
| D | Water bath 37°C | 15.18 | 146.00 |

As the measuring results show, the strips treated with NMP, i.e. the strips that in addition to the matrix component comprise a plasticizer, were considerably more flexible than the untreated strips. In other words, a membrane treated with NMP is substantially easier to work into the desired shape than an untreated membrane.

In example 1, the NMP content of the material is approximately 45 percent by weight from the total mass of NMP and the matrix material. When inserted into the organ system, the NMP content of the implant of the invention is 20 to 55, preferably 30 to 50, most preferably 40 to 45 percent by weight.

When the strips treated with NMP were kept in a water bath for 24 hours, the obtained yield strength and tensile modulus values were approximately two times higher than the corresponding values in the samples that were stored and tested in indoor air. This is due to the fact that water makes NMP slowly dissolve from the membrane and consequently, the structure of the membrane becomes more rigid. The same happens when the membrane is inserted into the organ system: NMP dissolves in tissue fluids and the membrane hardens to achieve a sufficient rigidity to support the tissues in the intended manner.

### Example 2

In a second preferred embodiment of the method of the invention, NMP is already added to the rest of the implant material when the implant is being made. Thus, trimethylenecarbonate/polylactide copolymer TMC/PLA (10:90) in melt form was mixed in an extruder with NMP in such a manner that in the resulting material, the NMP proportion was 30 percent by weight. A 0.3-mm thick membrane was extruded from the material. Tensile test pieces were made from the membrane and the testing was conducted according to the SFS-ISO 1184 standard.

The following test values were obtained for the material: tensile modulus 34.6 MPa, yield strength 3.2 MPa and tensile strength at break 13.6 MPa. When comparing the tensile modulus and yield modulus with the corresponding values obtained in example 1 and presented in table 1, it can be seen that they are in the same range. Thus, the time when NMP and the matrix polymer are mixed bears no essential significance to the properties of use of the membrane.

### Example 3

As done in example 1, a 0.2-thick membrane was made of poly(L-lactide-co-trimethylencarbonate-co-polyglycolide) copolymer PLLA/PGA/TMC (80:10:10). 20 strips of 5 x 30 mm were cut from the membrane and further treated by immersing them in an NMP solution for 30 seconds. The strips were allowed to homogenize for NMP to diffuse for 20 minutes. The strips were divided into a first and a second set. The 10 strips of the first set were tested at indoor temperature with a pulling machine at a pulling rate of 20 mm/min with the pulling distance at 10 mm initially. The 10 strips of the second set were immersed in a phosphate buffer solution and kept there at a temperature of 37°C for 24 hours before testing in a water bath of 37°C.

Strips of 5 x 30 mm and having a thickness of 0.3 mm were cut from three GTR membranes on the market and manufactured by W.L. Gore & Associates, Inc. for a tensile test. Two sets of four tensile test samples were made, and the samples of the first set were tested as such at indoor temperature. The samples of the second set were immersed in a phosphate buffer solution at a temperature of 37°C for 24 hours before testing. The samples of the first set were measured in a water bath of 37°C. All measurements were made according to the SFS-ISO 1184 standard. Table 2 shows the results of the tensile tests in Newton [N].

**Table 2:**

| Averages of the tensile test results of example 3 | | |
|---|---|---|
| Membrane type | Yield strength [N] Set I | Yield strength [N] Set II |
| PLLA/PGA/TMC | 4.1 | 14.5 |
| GTR Resolut | 5.2 | 4.7 |
| GTR OsseoQuest | 5.7 | 2.5 |
| GoreTex | 8.8 | 2.2 |

On the basis of the obtained tensile test results, it can be noted that the PLLA/PGA/TMC membrane treated with NMP has a yield strength value that corresponds to the membrane products on the market. In the phosphate buffer solution, the PLLA/PGA/TMC membrane treated with NMP becomes harder due to the diffusion of NMP in such a manner that its yield strength value is approximately 3 to 7 times higher than the yield strength values of the corresponding membranes on the market. In other words, the person performing the operation can easily shape the membrane of the invention to fit in the best possible manner to the organ system, and when the membrane is inserted into the organ system, its rigidity increases substantially as NMP diffuses out, whereby the membrane retains its shape under the pressure caused by the tissues.

### Example 4

A 0.3-mm thick polymer membrane was made of polymer granulates directly by compression pressing. The used polymers and the compression pressing parameters are shown in table 3.

**Table 3:**

| Polymer materials and processing parameters of example 4 | | | |
|---|---|---|---|
| Material | Maximum temperature [°C] pressing | Maximum pressure [bar] pressing | |
| PLLA | 200 | 120 | |
| PLLA/TMC 70:30 | 160 | 120 | |
| PLLA/PGA 50:50 | 165 | 120 | |

Strips of 40 x 5 mm were made of the obtained membranes for tensile tests. Two sets of ten samples were made of each material. The samples were immersed in an NMP solution for 40 seconds, after which the samples were lifted on top of a metal net for 30 minutes to ensure the diffusion of NMP to the polymer.

The samples of the first sets were tested at indoor temperature without any special pre-treatment. The samples of the second sets were immersed in a water bath of 37°C for 24 hours. The tensile test of the samples belonging to the first sets was conducted at indoor temperature in the atmosphere and the tensile test of the samples of the second sets was conducted in a water bath of 37°C. The samples were tested with an Instron material testing device. The testing was conducted according to the SFS-ISO 1184 standard at a pulling rate of 20 mm/min with the distance between the sample holders at 10 mm initially. The results of the tensile tests are shown in table 4.

**Table 4:**

| Averages and deviations of the tensile test results of example 4 | | | | |
|---|---|---|---|---|
| Material | Set | Yield strength [MPa] | Tensile strength [MPa] | Tensile modulus [MPa] |
| PLLA | I RT | 4.68 | 6.48 | 23.16 |
| PLLA | II 37°C | 8.75 | 8.84 | 117.84 |
| PLLA/TMC | I RT | 0.18 | 0.34 | 1.93 |
| PLLA/TMC | II 37°C | 1.26 | 1.95 | 12.40 |
| PLLA/PGA | I RT | 1.27 | 1.78 | 8.67 |
| PLLA/PGA | II 37°C | 2.61 | 2.71 | 51.54 |

The results in table 4 show that the tensile modulus values of the samples kept and measured in the atmosphere, i.e. of the first sets, are considerably lower than those of the corresponding samples of the second sets that were kept in a phosphate buffer fluid for 24 hours. Similarly, the yield and tensile strength values of the first sets are lower than those of the corresponding samples of the second sets.

NMP reduces the rigidity of the membrane so that the membrane can easily be shaped as desired. When NMP in a membrane inserted in tissue reacts with water originating from the tissues, the rigidity of the membrane increases substantially and may even return to the original state that prevailed prior to adding the plasticizer. This way, the membrane of the invention can be made so thick that its rigidity is sufficient to support tissue during regeneration and yet the membrane can very easily be shaped as desired at the shaping stage.

The drawings and the related specification and the examples are only intended to illustrate the idea of the invention. The invention may vary in detail within the scope of the claims. Thus, the implant can also be a three-dimensional spatial piece, a fixing element, such as screw, pin or suture, or any other shaped-piece implant known per se. Active agents, such as antibiotics, pharmaceutical products, growth hormones, styptic agents, chemotherapy agents or their precursors and the like that diffuse from the implant in a controlled manner to the surrounding tissue, can be added to the implant. For instance, the article "Preliminary *In Vivo* Studies on the Osteogenic Potential of Bone Morphogenetic Proteins Delivered from an Absorbable Puttylike Polymer Matrix" discloses a puttylike polymer matrix having the proteins (BMP, Bone Morphogenetic Protein) affecting bone regeneration added to it diffuse in a controlled manner into the surrounding tissue. Proteins affecting bone regeneration of this kind can easily be added to the implant of the invention. The active agents can be added to the implant for instance by dissolving them first in a plasticizer, such as NMP, and then treating the polymer matrix component with a solution comprising the solvent and the active agent.

## Claims

1. A biodegradable implant comprising:
a matrix component containing at least one biodegradable polymer or copolymer, and
a plasticizer that is adapted to reduce substantially the rigidity of the implant,
which plasticizer substantially exits from the implant after coming into contact with tissue fluids of the organ system in such a manner that
the bending resistance of the implant prior to the insertion of the implant into the organ system is substantially lower than after its insertion into the organ system.

2. A biodegradable implant comprising:
a matrix component containing at least one biodegradable polymer or copolymer, and
a plasticizer that is adapted to reduce substantially the rigidity of the implant,
which plasticizer substantially comprises N-methyl-2-pyrrolidone (NMP),
and which plasticizer substantially exits from the implant after coming into contact with tissue fluids of the organ system in such a manner that
- the bending resistance of the implant prior to the insertion of the implant into the organ system is substantially lower than after its insertion into the organ system.

3. An implant as claimed in claim 1 or 2, wherein the matrix component comprises at least one of the following polymers or copolymers that is selected from the following group: polyglycolide, polylactides, polycaprolactones, polytrimethylenecarbonates, polyhydroxybutyrates, polyhydroxyvalerates, polydioxanones, polyorthoesters, polycarbonates, polytyrosinecarbonates, polyorthocarbonates, polyalkylene oxalates, polyalkylene succinates, poly(malic acid), poly(maleic anhydride), polypeptides, polydepsipeptides, polyvinylalcohol, polyesteramides, polyamides, polyanhydrides, polyurethanes, polyphosphazenes, polycyanoacrylates, polyfumarates, poly(amino acids), modified polysaccharides, modified proteins and copolymers thereof.

4. An implant as claimed in any one of the claims 1 to 3, wherein at least the surface of the implant is porous.

5. An implant as claimed in any one of the claims 1 to 4, wherein active agents, such as antibiotics, pharmaceutical products, growth hormones, styptic agents, chemotherapy agents, are arranged in the implant.

6. An implant as claimed in any one of the claims 1 to 5, wherein the plasticizer is added to the matrix material at the latest at the forming stage of the implant.

7. An implant as claimed in any one of the claims 1 to 5, wherein the plasticizer is added to the implant just before the implant is inserted into the organ system.

8. An implant as claimed in any one of the claims 1 to 7, wherein the implant is a membrane used in guided tissue regeneration.

9. A method for manufacturing a biodegradable implant comprising:
selecting biodegradable polymer(s) or copolymer(s) of a matrix component of the implant,
adding a plasticizer to the matrix component,
which plasticizer substantially exits from the implant after coming into contact with tissue fluids of the organ system in such a manner that the rigidity of the implant increases substantially after the implant is inserted into the organ system, and
forming the implant from the mixture of said matrix component and plasticizer.

10. A method for manufacturing a biodegradable implant comprising:
selecting biodegradable polymer(s) or copolymer(s) of a matrix component of the implant,
forming the implant from said matrix component, and
adding a plasticizer to the matrix component,
which plasticizer substantially exits from the implant after coming into contact with tissue fluids of the organ system in such a manner that the rigidity of the implant increases substantially after the implant is inserted into the organ system.

11. A method as claimed in claim 9 or 10, wherein the plasticizer comprises N-methyl-2-pyrrolidone (NMP).

12. A method as claimed in any one of the claims 9 to 11, wherein the plasticizer is added to the implant just before the implant is inserted into the organ system.

13. A method as claimed in any one of the claims 9 to 12, wherein the matrix component comprises at least one of the following polymers or copolymers that is selected from the following group: polyglycolide, polylactides, polycaprolactones, polytrimethylenecarbonates, polyhydroxybutyrates, polyhydroxyvalerates, polydioxanones, polyorthoesters, polycarbonates, polytyrosinecarbonates, polyorthocarbonates, polyalkylene oxalates, polyalkylene succinates, poly(malic acid), poly(maleic anhydride), polypeptides, polydepsipeptides, polyvinylalcohol, polyesteramides, polyamides, polyanhydrides, polyurethanes, polyphosphazenes, polycyanoacrylates, polyfumarates, poly(amino acids), modified polysaccharides, modified proteins and copolymers thereof.

14. A method as claimed in any one of the claims 9 to 13, wherein the implant is porous.

15. A method as claimed in any one of the claims 9 to 14, wherein active agents are added to the implant.

16. A method as claimed in claim 15, wherein the active agents are first mixed into the plasticizer and then added together with the plasticizer to the matrix component.

17. A method as claimed in any one of the claims 9 to 16, wherein the implant is a membrane used in guided tissue regeneration.
